# EUROPEAN PATENT SPECIFICATION

(11) **EP 4 107 237 B1**
(45) Date of publication and mention of the grant of the patent: **19.02.2025**
(21) Application number: 21756576.1
(22) Date of filing: 25.01.2021
(51) Int. Cl.: C10L 5/44, B01D 3/14, B01D 5/00, C07D 307/50, D21B 1/36, D21F 5/20

(54) **RECOVERY OF ENERGY AND CHEMICALS FROM A STEAM EXPLOSION PROCESS**
RÜCKGEWINNUNG VON ENERGIE UND CHEMIKALIEN AUS EINEM DAMPFEXPLOSIONSVERFAHREN
RÉCUPÉRATION D'ÉNERGIE ET DE PRODUITS CHIMIQUES ÉMANANT D'UN VAPOCRAQUAGE

(30) Priority: 20.02.2020 SE 2050186
(43) Date of publication of application: 28.12.2022
(73) Proprietor: VALMET AB, 85 194 Sundsvall (SE)
(72) Inventor: BJÖRKLUND, Peter, 907 37 Umeå (SE)
(74) Representative: Novagraaf Group
(86) International application number: PCT/SE2021/050039
(87) International publication number: WO 2021/167511

(56) References cited:
- WO-A1-2016/020269
- WO-A1-2018/104485
- WO-A1-2018/122454
- WO-A1-2019/093938
- WO-A1-93/15816
- No further relevant documents disclosed

## Description

### TECHNICAL FIELD OF THE INVENTION

The present invention relates to a method and a system for recovery of energy and chemicals from a steam explosion plant for production of biomass pellets or briquettes. It further relates to a method and a system for recovery of energy and chemicals from a steam explosion reactor in a biorefinery.

### BACKGROUND

It is known art to process a lignocellulosic biomass such as wood chips with steam at elevated temperature in a pressurised reactor for softening of lignin and release of hemicellulose sugar by hydrolysis. Such pressure heating may be followed by a rapid discharge of the heated biomass through a valve or an orifice to low (often atmospheric) pressure. The hot and soft biomass defibrates to small particles during the rapid and violent discharge providing a substrate, excellent for pressing to fibre-board or other mouldable products or alike. Such thermal heating of a biomass in a pressurised reactor followed by rapid discharge to low pressure is in the following called "steam explosion" or "steam explosion method". The steam explosion method may also be used as a pre-treatment step at production of durable and moisture resistant fuel pellets or briquettes from various lignocellulosic biomasses. Biomass of woody origin, in the form of chips or flakes, for example wood chips, bark, saw-dust or agricultural residues, for example straw, bagasse, empty fruit bunches and like are charged to a reactor which is pressurised with steam. Condensing steam heats up the biomass which is, after a beneficial treatment time in the reactor, discharged through a blow valve to a receiving container, in the following called a "blow-tank". The steam exploded biomass is thereafter densified to pellets or briquettes which can replace fossil coal in heat and power generation. Pellets or briquettes produced from biomass are of great interest as they are sustainable and green-house gas neutral fuel.

The steam explosion method is a thermal method, biomass is heated with steam, which is the main consumable. A large amount waste steam, in the following called "blow-steam" is released from the mixture of steam and hot moist biomass which is discharged from the reactor to the blow tank. The blow-steam is separated from biomass in the blow-tank or with a separator, for example a cyclone or a centrifuge, installed upstream of the blow-tank. Blow-steam, withdrawn from the blow-tank or a separator, is not pure water steam. It is contaminated with numerous organic compounds, formed through chemical reactions during the thermal treatment and which are released partly to vapour phase during reactor discharging. The compounds, for example acetic acid, acetone, formic acid, methanol, furfural and alike, are considered as pollutants and cannot be release to atmosphere. But, the compounds may in some cases have a high market value. For example, furfural is used as such or refined to solvents, fuel, resin, fungicides, nematicides and other. It is of great interest to improve the economic feasibility and environmental performance at production of densified steam exploded fuel. Recovery volatile hydrocarbons from blow-steam to saleable products will bring additional revenues to the pellet plant operator.

Steam explosion methods were originally developed for production of wood fibre mainly for board. Both discontinuous (batch) and continuous methods are known. An early discontinuous method is disclosed in US1578609 which describes an intermittently operated reactor, a "gun", for production of wood fibre. Wood chips are charged to a reactor which is sealed with valves and pressurised to 19-40 bar by injecting for example direct steam. The gun is then discharged and depressurised by opening a valve to a blow-tank from which the steam exploded fibre is supplied for further treatment to board. Discontinuous methods for production of fuel pellets with the steam explosion method are disclosed in US7303707, NO327839 and NO320971. Said methods teach us to process for example wood chips charged into a reactor with steam under pressure for a beneficial duration and then rapidly discharging the reactor to lower pressure. Common to these known methods is that the pressure prevailing in the reactor is not constant but returned to ambient in one or several steps. The discontinuous operation mode results in that the blow-steam flow from the process vary enormously and consequently is recovery of heat and chemicals from blow-steam challenging. Application NO20130279 discloses a method for improving recovery of energy from discontinuous steam explosion processes. In this process is a blow-steam condenser followed by a container with a flexible volume for equalizing the vapour volume flow. Said methods, US7303707, NO327839 and NO320971, teach also to reduce use of steam by integrating varying drying concepts to the steam explosion step, such as pre-drying for reduction of steam usage in the steam explosion reactor or a combination of pre and post drying for reduce of energy for drying.

Difficulties, inter alia at recovery of energy at steam explosion induced development of a continuous steam explosion method. Such continuous methods are disclosed for example in US1922313 and US2616802. US1922313 discusses the difficulty of recovery of steam from discontinuous steam explosion guns and discloses a continuously operated steam explosion method. These methods teach to feed wood chips with a continuously operated, pressure sealing screw, to a reactor where chips are heated to the processing temperature with direct steam. The reactor is horizontal and the chips transportation through the screw is facilitated by means of an auger. The heated chips exit the reactor continuously through a blow-valve located below the reactor. The reactor discharge is by "steam explosion method" since there is a high pressure-drop over the blow-valve. Heat recovery from blow-steam is technically greatly improved with the continuous process since the reactor pressure and the pressure drop over the blow valve is constant, resulting in a continuous flow of blow-steam. Said art is intended for steam explosion of moist wood. US1922313 teaches to furnish the pressure sealing screw with draining holes, so dewatering some water from the wood chips and equalizing the moisture content of them. It is known that this type of screws may dewater wood chips only to around 45 % of moisture and this means that still a significant amount of water must be heated with steam in the continuous process of US1922313.

It is noted with regards to the furfural in blow-steam that known art includes many methods for production of it from corn cobs, bagasse, hardwoods and other lignocellulosic biomasses rich in pentosane. An overview of continuous and discontinuous methods for production of furfural is given by K.J. Zeitsch in his book "The chemistry and technology of furfural and its many by-products" chapter 10 pages 36-75. Typically, is a lignocellulosic biomass rich in pentosane acidulated with a mineral acid and treated under elevated temperature and pressure. Pentosan is consecutively hydrolysed and dehydrated to furfural which is stripped from the moist biomass with steam. The feasibility of such known furfural processes is burden by high operational expenditure for heating and stripping steam. Zeitsch teach in DE19905655 that present processes for furfural are extremely expensive to operate, the steam requirement is in the range 30-50 ton per ton of furfural produced. DE19905655 teach a method for reduced steam usage and improved furfural yield, but there is still an opportunity for alternative methods for furfural recovery with low operational expenditure.

A method for recovery of heat from blow-steam from a continuously operated steam explosion reactor is disclosed in SE541264. Said method teach passing blow-steam to a boiler furnace, then recovering the condensing heat from the flue gases of said boiler, further transferring recovered heat to a biomass dryer. SE541264 resolves an air emission problem as well, since volatile hydrocarbons in blow-steam are efficiently combusted in the boiler furnace.

WO2018122454A1 discloses a method for recovery of energy from a continuously operated steam explosion reactor for production of biomass, said method comprising the steps of: condensing a blow-steam from the steam explosion reactor for receiving a blow-steam condensate; recovering heat from said condensation of the blow-steam to energise a fractionation system, in which fractionation system volatile hydrocarbon compounds are recovered from said blow-steam condensate.

### SUMMARY

An object of the invention is to recover energy of a blow-steam from a steam explosion reactor.

A further object of the invention is to improve recovery of furfural from a blow-steam from a steam explosion reactor.

These objects are achieved in a method and an energy recovery system according to the independent claims.

According to one aspect of the invention a method is provided for recovery of energy from a continuously operated steam explosion reactor for production of biomass pellets or briquettes, said method comprising the steps of:
- condensing a blow-steam from the steam explosion reactor for receiving a blow-steam condensate;
- recovering heat from said condensation of the blow-steam to energise a fractionation system, in which fractionation system volatile hydrocarbon compounds are recovered from said blow-steam condensate; and
- recovering heat from said fractionation system (300b) for drying of biomass used in the steam explosion reactor.

According to another aspect of the invention method is provided for recovery of energy from a continuously operated steam explosion reactor provided in a biorefinery, said method comprising the steps of:
- condensing a blow-steam from the steam explosion reactor for receiving a blow-steam condensate;
- recovering heat from said condensation of the blow-steam to energise a fractionation system, in which fractionation system volatile hydrocarbon compounds are recovered from said blow-steam condensate; and
- recovering heat from said fractionation system for heating process fluids in the biorefinery.

According to another aspect of the invention an energy recovery system is provided for recovery of energy from a continuously operated steam explosion reactor for production of biomass pellets or briquettes, said energy recovery system being configured to be connected to a steam explosion plant for receiving a blow-steam (b) from a steam explosion reactor provided in said steam explosion plant, said energy recovery system comprising:
- a reboiler system configured to receive said blow-steam (b) from the steam explosion reactor, wherein said reboiler system comprises a first reboiler which is configured to condense the blow-steam into a blow-steam condensate comprising water and a mixture of volatile hydrocarbon compounds comprising furfural, whereby a reboiled steam is raised in the first reboiler during condensation;
- a fractionation system connected to the reboiler system and configured for receiving the blow-steam condensate and the reboiled steam from the reboiler system, wherein the fractionation system further is configured to use energy from the reboiled steam for recovery of furfural from the blow-steam condensate, whereby an overhead vapour is obtained in the fractionation system during recovery of furfural; and
- a reflux condensing system connected to the fractionation system and configured for receiving the overhead vapour from the fractionation system, wherein said reflux condensing system further is connected to a biomass dryer provided in the steam explosion plant and wherein the reflux condensing system is configured to recover energy from the overhead vapour and use this energy for heating a heat transfer fluid (i) used in the dryer for drying biomass used in the steam explosion reactor.

According to another aspect of the invention an energy recovery system is provided for recovery of energy from a continuously operated steam explosion reactor, said energy recovery system being configured to be connected to a biorefinery for receiving a blow-steam (b) from a steam explosion reactor provided in said biorefinery, said energy recovery system comprising:
- a reboiler system configured to receive said blow-steam (b) from the steam explosion reactor, wherein said reboiler system comprises a first reboiler which is configured to condense the blow-steam into a blow-steam condensate comprising water and a mixture of volatile hydrocarbon compounds comprising furfural, whereby a reboiled steam is raised in the first reboiler during condensation;
- a fractionation system connected to the reboiler system and configured for receiving the blow-steam condensate and the reboiled steam from the reboiler system, wherein the fractionation system further is configured to use energy from the reboiled steam for recovery of furfural from the blow-steam condensate, whereby an overhead vapour is obtained in the fractionation system during recovery of furfural; and
- a reflux condensing system connected to the fractionation system and configured for receiving the overhead vapour from the fractionation system, wherein said reflux condensing system further is connected to the biorefinery and wherein the reflux condensing system is configured to recover energy from the overhead vapour and use this energy for heating process fluids in the biorefinery.

Hereby a method and a system are achieved which effectively utilize the energy-content of the blow-steam and which effectively recover saleable chemical by-products in the blow-steam, such as furfural, at low cost. Energy from the blow-steam itself is used in the recovery of furfural in the fractionation system. Recovered energy from the fractionation system is utilized for drying of biomass which is provided into the steam explosion plant for production of biomass pellets or briquettes or alternatively for heating of process fluids in the biorefinery. Hereby energy from the blow-steam is used both for recovery of furfural and for drying of biomass or for heating of process fluids in a biorefinery. The waste energy in the blow steam is used very efficiently as it is used twice.

Suitable embodiments of the invention are described in the dependent claims and in the detailed description below.

### BRIEF DESCRIPTION OF THE DRAWINGS

Figure 1 schematically exemplifies a steam explosion plant for production of biomass pellets or briquettes to which the present invention can be applied.
Figure 2 is a schematic and general overview summarizing features and flows of materials and energy of the present invention.
Figure 3a is a schematic representation of an energy recovery system according to one embodiment of the invention.
Figure 3b is a schematic representation of an energy recovery system according to another embodiment of the invention.
Figure 4 is a flow chart of a method according to one embodiment of the invention.

### DETAILED DESCRIPTION OF THE INVENTION

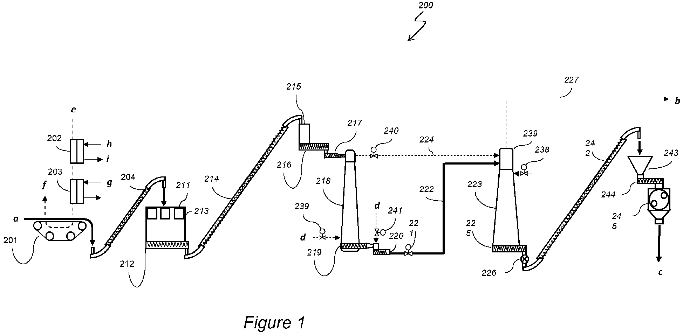

Figure 1 shows schematically a steam explosion plant 200 for production of steam exploded and densified fuel from a lignocellulosic biomass for example wood, bark, bagasse and straw, and like. The wet biomass (a), is split up to micro-chips, flakes or splinters and it may have been screened for removal of unwanted components such as sand or gravel which can cause wear and blockages in downstream machineries. The biomass is next dried with a dryer 201, for example a low temperature belt dryer, for example to the range 6-12 % to reduce the use of steam (d) for heating in a steam explosion reactor 218 of the steam explosion plant 200. In the example of Figure 1 drying air (e) which is supplied to the dryer 201 is heated in two steps, first with a pre-heater 202 and then with steam coils 203. The heated air dries the microchips and exits as humid air to atmosphere (f). The dried biomass can be fed with a conveyor, 204, to a receiving bin 211 which equalises variations in raw material flow and provides a sufficient volume for a stable material supply to the steam explosion reactor 218. The biomass is, as said preferably dry, but the process works also with moist raw-materials, though at the expense of a higher steam consumption (d) since more water must be heated in the reactor 218. The receiving bin 211 is in this example emptied from the bottom with screws 212 and/or scrapers to a conveyor 214 which transports the biomass to a level bin 215. The level bin is furnished with a chip level control which operates the upstream screw 212 and the conveyor 214. The level bin 215 is operated at atmospheric pressure but the reactor 218 operates at elevated temperature, 170-215 °C, and pressure, 10-25 bar(a). Two screws, a first screw 216 and a second screw 217, may be provided for transferring the biomass from the level bin 215 to the reactor 218. The first screw 216, which is operated essentially at atmospheric pressure, provides a constant flow of biomass to the second screw 217. The second screw 217, also denoted "plug-screw", is conical and it compresses the biomass to a hard plug such that the high pressure into the reactor 218 is sealed to atmospheric pressure. Both screws work continuously and the hard pressure sealing biomass plug formed in the second screw 217 is mechanically disintegrated when it exits into the reactor 218. Steam (d), is added to the reactor for heating and hydrolysing the biomass. Hemicelluloses of the biomass are easily hydrolysed at the high temperature prevailing in the reactor forming sugar oligomers and monomers. A part of the sugars released will dehydrate to furfural and hydroxymethyl furfural. Also, other volatile by-products are formed and released from the biomass, such as acetic acid, formic acid and methanol. Biomass is emptied from the reactor 218 for example with a screw and/or a scraper 219 which is installed in the bottom. The steam explosion reactor 218 discharges to a blow tank 223 through a blow valve 221 with an adjustable aperture or alternatively through a fixed orifice. The pressure-drop over blow valve 221 is very high since the upstream pressure is 10-25 bar and the pressure in the blow-tank is considerably lower. The high pressure-drop over the blow valve results in steam explosion so that water evaporates in the pores and cavities of the biomass when pressure rapidly decreases, thereby expanding and defibrating the biomass. The result of steam explosion is an open and disintegrated material structure suitable for densification to pellets or briquettes. The blow tank, 223, is preferably operated close to atmospheric pressure, 1,01 bar(a), but it is possible to operate it at overpressure, for example up to 6 bar(a), without jeopardising the steam explosion effect over the blow valve 221. A rotating mixer or screw, 220, may be installed upstream the blow-valve 221, for assisting of the discharge of biomass through the blow valve 221 or a discharge orifice. Further, steam (d) may be added to said mixer, 220, for helping discharge of biomass through the blow valve 221. The mixture of steam originating from the reactor 218 and steam obtained from water and volatile hydrocarbons which evaporate from moist hydrolysed biomass is blow-steam. Hydrolysed and steam exploded biomass is collected in the blow tank, 223 which is sealed to ambient atmosphere such that air cannot leak into it. Steam exploded biomass and steam are separated with a cyclonic part 239 installed at the top of the blow tank 223. The biomass inlet to the blow tank is tangential and the diameter of the top part is selected such that a high tangential velocity is reached for good separation of steam and biomass. Steam and steam exploded biomass may be alternatively separated for example with a cyclone or a centrifuge (not shown in figure) upstream the blow-tank. Steam from the cyclone or centrifuge is transferred to recovery and the steam exploded biomass to the blow-tank.

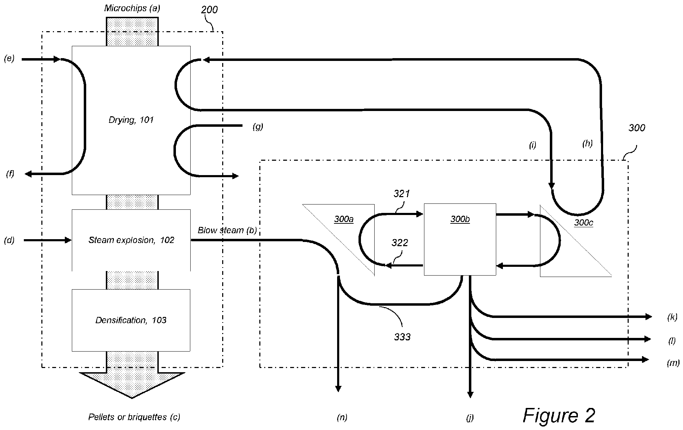

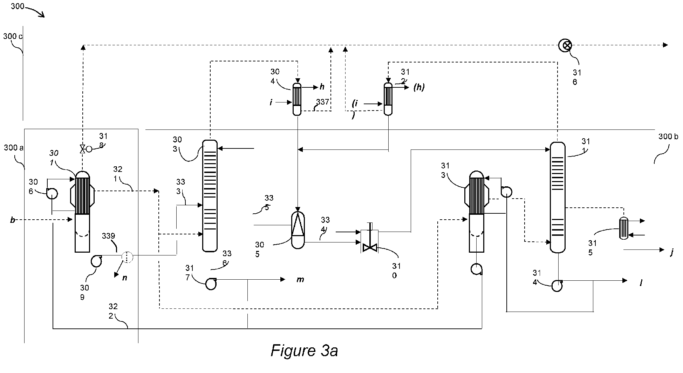

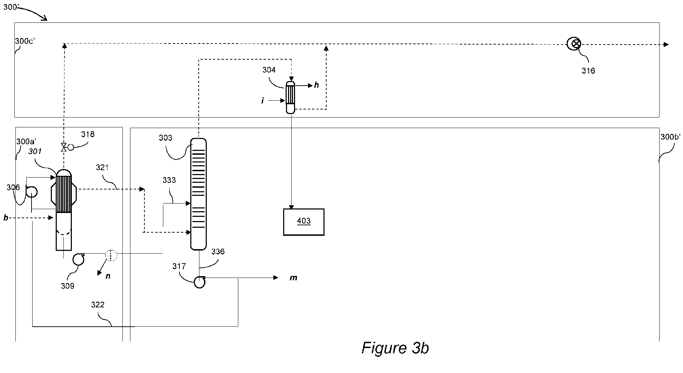

According to the invention, blow-steam is conducted with a pipe 227 from the blow-tank 223 (or a cyclone or centrifuge if used) to an energy recovery system 300 which will be further described below in relation to Figures 2 and 3. The pressure in the blow tank 223 is controlled with the energy recovery system 300. Volatile compounds and inert gas (air) may be released from the reactor 218 by opening a relief valve 240 to a relief pipe 224 which conducts the vapour mixture to the blow tank 223. The steam usage in the reactor 218, which depends on the moisture content of the biomass is 0,40-0,65 ton for each ton of biomass charged (bone dry basis). Most of the steam (d) exits the reactor through the valve 221 mixed with the steam exploded biomass, a part of it is consumed for heating of the lignocellulosic biomass and the water contained therein, a small amount of it compensates for the volatiles and inert gases released out through the relief valve 240. The quality of the steam exploded product (c) is largely determined by the duration and the temperature in the reactor 218. The duration of the biomass in the reactor at a certain production capacity is determined by controlling the biomass level in the reactor and it is typically up to 20 minutes. The temperature in the reactor is as said in the range 170-215 °C and it is determined by the pressure control in the reactor. These variables are fixed for the selected product quality and they are kept constant during processing, i.e. the biomass level, temperature and the pressure in the reactor are maintained constant at a certain production capacity. Steam exploded biomass from the blow-tank 223 can be transported with a conveyor 242 to a silo or like 243, from which the biomass is distributed with conveyors 244, to pelleting or briquetting machines 245. The operation model of the described steam explosion reactor is continuous. The set point for the reactor pressure is constant providing a constant pressure drop over valve 221 and a continuous steam flow to the blow tank and further to the energy recovery system 300. The present invention is targeting and applicable for a such continuous steam explosion reactor system 200 as described in relation to Figure 1 but also for other similar systems, such as a biorefinery.

The method and system according to the invention for recovery of energy and furfural from a steam explosion process is schematically disclosed in Figure 2. The schematics of Figure 2 is split in two distinguished areas, denoted 200 and 300. Area 200 comprises a typical process for production of steam exploded pellets or briquettes and area 300 comprises a process for recovery of energy and furfural from blow-steam withdrawn from steam explosion. Figure 2 does not include details of the processes, the purpose of it is only to schematically explain principles behind the present method. Parts of the energy recovery system 300 according to the invention are only schematically shown in relation to each other in Figure 2. Further details of the energy recovery system are given in Figures 3a and 3b which will be described in detail below. The area 200 in Figure 2 will be modified for those embodiments of the invention where the method and system are applied for a biorefinery instead of a plant for production of pellets or briquettes. The drying 101 and the densification 103 are replaced by other method steps provided in the biorefinery. The steam explosion, the transfer of the blow-steam and the area 300 are however the same also for these embodiments.

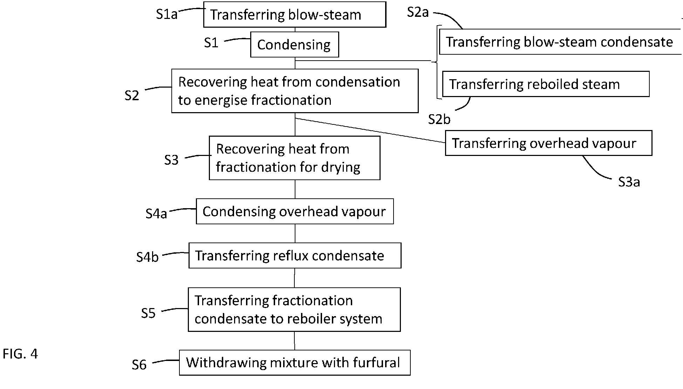

According to the invention a method is provided for recovery of energy from a continuously operated steam explosion reactor 218 for production of biomass pellets or briquettes. The steps of the method are described below and are shown in the flow chart in Figure 4:
S1: Condensing a blow-steam from the steam explosion reactor 218 for receiving a blow-steam condensate.
S2: Recovering heat from said condensation of the blow-steam to energise a fractionation system 300b, in which fractionation system volatile hydrocarbon compounds are recovered from said blow-steam condensate.
S3: Recovering heat from said fractionation system 300b for drying of biomass used in the steam explosion reactor 218.

The energy recovery system 300 according to the invention is configured to be connected to a steam explosion plant 200 for receiving a blow-steam (b) from a steam explosion reactor 218 provided in the steam explosion plant 200. The energy recovery system 300 comprises a reboiler system 300a configured to receive said blow-steam (b) from the steam explosion reactor 218, wherein said reboiler system 300a comprises a first reboiler 301 which is configured to condense the blow-steam into a blow-steam condensate comprising water and a mixture of volatile hydrocarbon compounds comprising furfural, whereby a reboiled steam is raised in the first reboiler 301 during condensation. The energy recovery system 300 comprises furthermore a fractionation system 300b connected to the reboiler system 300a and configured for receiving the blow-steam condensate and the reboiled steam from the reboiler system 300a, wherein the fractionation system 300b further is configured to use energy from the reboiled steam for recovery of furfural from the blow-steam condensate, whereby an overhead vapour is obtained in the fractionation system 300b during recovery of furfural. The energy recovery system 300 comprises also a reflux condensing system 300c connected to the fractionation system 300b and configured for receiving the overhead vapour from the fractionation system 300b, wherein said reflux condensing system 300c further is connected to a biomass dryer 201 provided in the steam explosion plant 200 and wherein the reflux condensing system 300c is configured to recover energy from the overhead vapour and use this energy for heating a heat transfer fluid (i) used in the dryer 201 for drying biomass used in the steam explosion reactor 218. The reboiler system 300a, the fractionation system 300b and the reflux condensing system 300b are shown in more detail in Figures 3a and 3b.

The step of condensing S1 a blow-steam is hereby performed in a reboiler system 300a whereby a blow-steam condensate is achieved in the reboiler system 300a and a reboiled steam is raised in the reboiler system 300a. The step S2 of recovering heat from said condensation to energise a fractionation system 300b comprises using said reboiled steam to energise said fractionation system 300b and the step S3 of recovering heat from said fractionation system 300b for drying of biomass comprises transferring an overhead vapour which is obtained in said fractionation system 300b during recovery of volatile hydrocarbon compounds and using said overhead vapour in a reflux condensation system 300c which is connected to the fractionation system 300b for heating of a heat transfer fluid for drying of the biomass.

The method according to the invention is now described in more detail. An initial step performed before the step of condensing S1 is a step of transferring S1a a blow-steam (b) from a steam explosion plant 200 for production of biomass pellets or briquettes to a reboiler system 300a. The blow-steam (b) can be transferred from a blow tank 223 which is provided in the steam explosion plant 200. The step of condensing S1 comprises condensing the blow-steam in the reboiler system 300a for receiving a blow-steam condensate comprising water and a mixture of volatile hydrocarbon compounds comprising furfural. The next two steps are one step of transferring the blow-steam condensate S2a to a fractionation system 300b and a step of transferring a reboiled steam S2b which is raised in the reboiler system 300a during condensation from the reboiler system 300a to the fractionation system 300b. Then the reboiled steam is used to energise the fractionation system 300b for recovery of furfural. This is step S2, i.e. recovering heat from said condensation of the blow-steam to energise a fractionation system 300b as described above. Next an overhead vapour which is obtained in the fractionation system 300b during fractionation is transferred S3a to a reflux condensing system 300c and then a heat transfer fluid (i) in the reflux condensing system 300c is heated for use in a pre-heater 202 for heating of a drying gas to a biomass dryer 201 provided in the steam explosion plant 200 for drying biomass, wherein said heating is performed by energy recovered in the reflux condensing system 300c. This is step S3 as described above.

In some embodiments a further step of condensing S4a in the reflux condensing system 300c the overhead vapour to a reflux condensate comprising furfural is performed. Furthermore, a step of transferring S4b said reflux condensate back to the fractionation system 300b for further recovery of furfural is performed in some embodiments.

In some embodiments a further step of transferring a fractionation condensate S5 from the fractionation system 300b to the reboiler system 300b to be used as feed water in the reboiler system 300b is performed. Said fractionation condensate is achieved in the fractionation system during recovery of furfural.

The method may further comprise the step of withdrawing S6 a liquefied mixture comprising furfural (j) from the fractionation system 300b, wherein the liquefied mixture (j) comprises at least 35% furfural or at least 75% furfural or at least 94% furfural.

The method and energy recovery system can be a method and a system for recovery of energy from a process for production of pellets or briquettes from lignocellulosic biomass in a steam explosion plant 200, wherein treatment of the biomass is performed in a continuously operated reactor 218 provided in the steam explosion plant 200 under a pressure in the range 10-25 bar(a) such that the lignocellulosic biomass is heated to a temperature within 10 °C to the saturation temperature of water at the pressure prevailing in the reactor 218 and wherein the treated material and blow-steam is continuously discharged from the reactor 218 through a blow valve 221 to a blow tank 223 provided in the steam explosion plant 200.

The method and energy recovery system according to the invention can also in another embodiment be a method and a system for recovery of energy from a continuously operated steam explosion reactor provided in a biorefinery. The energy recovery system 300 is in this embodiment of the invention configured to be connected to a biorefinery for receiving a blow-steam (b) from a steam explosion reactor provided in said biorefinery. The energy recovery system 300 comprises in this embodiment:
- a reboiler system 300a configured to receive said blow-steam (b) from the steam explosion reactor, wherein said reboiler system 300a comprises a first reboiler 301 which is configured to condense the blow-steam into a blow-steam condensate comprising water and a mixture of volatile hydrocarbon compounds comprising furfural, whereby a reboiled steam is raised in the first reboiler 301 during condensation;
- a fractionation system 300b connected to the reboiler system 300a and configured for receiving the blow-steam condensate and the reboiled steam from the reboiler system 300a, wherein the fractionation system 300b further is configured to use energy from the reboiled steam for recovery of furfural from the blow-steam condensate, whereby an overhead vapour is obtained in the fractionation system 300b during recovery of furfural; and
- a reflux condensing system 300c connected to the fractionation system 300b and configured for receiving the overhead vapour from the fractionation system 300b, wherein said reflux condensing system 300c further is connected to the biorefinery and wherein the reflux condensing system 300c is configured to recover energy from the overhead vapour and use this energy for heating process fluids in the biorefinery.

The method is in this embodiment of the invention is a method for recovery of energy from a continuously operated steam explosion reactor provided in a biorefinery, said method comprising the steps of:
- condensing a blow-steam from the steam explosion reactor for receiving a blow-steam condensate;
- recovering heat from said condensation of the blow-steam to energise a fractionation system, in which fractionation system volatile hydrocarbon compounds are recovered from said blow-steam condensate; and
- recovering heat from said fractionation system for heating process fluids in the biorefinery.

Recovery of furfural in the fractionation system 300b can for example comprise one or more of following methods: stripping, distillation, dehydration, decanting, reflux condensing and re-boiling bottoms of fractionation columns.

The reflux condensing system 300c comprises at least one reflux condenser 304 which is configured to receive said overhead vapour and condense the overhead vapour to a reflux condensate comprising furfural, wherein said reflux condensing system 300c in some embodiments further is configured for transferring said reflux condensate back to the fractionation system 300b for further recovery of furfural.

The fractionation system 300b can comprise one or more of an azeotropic distillation column 303 including a stripping and a rectifying section, a dehydration column 311, a decanter 305 and a reboiler 313.

In some embodiments of the invention said fractionation system 300b comprises at least one second reboiler 313 which is connected to and configured to energise a dehydration column 311 comprised in the fractionation system 300b. The second reboiler 313 may be connected to the first reboiler 301 and configured for utilising reboiled steam from said first reboiler 301 as heating fluid.

In some embodiments of the invention said first reboiler 301 is a shell and tube heat exchanger, for example a falling film evaporator. If one or more second reboilers 313 are provided in the fractionation system 300b these can also be shell and tube heat exchangers or falling film evaporators.

In some embodiments of the invention the fractionation system 300b can be configured to transfer a fractionation condensate from the fractionation system 300b to the reboiler system 300a to be used as feed water in the reboiler system 300a. Said fractionation condensate is achieved in the fractionation system during recovery of furfural.

Figure 2 is now described in more detail.

Production of steam exploded pellets or briquettes with a steam explosion process is now explained. A lignocellulosic biomass is milled or cut to small size chips or splinters, in the following called "microchips", (a). Microchips hold a natural moisture typically in the range 30-60 % and they are dried 101 to a residual moisture in the range 6-12 % before they are fed to thermal treatment in a steam explosion reactor. The drying 101 can be performed in a dryer 201 which may be a flash-dryer, drum dryer or low temperature belt-dryer, also other dryer types are possible. A drying gas (e) which may be air, is heated in heat-exchangers with steam (g) or a heating fluid (h) which may be hot water or a heat carrier comprising a mixture of ethylene-glycol or alike. The heated gas is used as drying medium in the biomass dryer, 201. Most of the energy for drying which is provided by heating fluids (g) and (h) exits the dryer as humidity in the exhaust gas (f). The dried microchips are fed to a pressurised reactor 218 where they are heated with direct condensing steam (d) close, within 10 °C, to the saturation temperature of water steam at the pressure prevailing, typically about to 200 °C. The biomass remains in the reactor, 218, up to 20 minutes and is thereafter discharged rapidly with a high pressure drop over a blow valve 221 or orifice to low (atmospheric) pressure. Part of the moisture within the biomass evaporates as steam, here denoted "blow-steam" (b) and the expanding steam disintegrates the structure of the hot and soft biomass. This type of biomass defibration over a high pressure-drop is also called "steam explosion" 102. The solid product from steam explosion is a fibrous pulp with suitable properties for densification, 103, to pellets or briquettes (c). According to the present invention energy is saved by recovery of the latent heat of the blow steam, (b), by first using it in a condensate fractionation process and then for heating of a heat carrier fluid (i) to the dryer, 201. Blow-steam condensate is contaminated with organic material, for example furfural, which has a market value. Furfural may be recovered from blow steam condensate with known fractionation methods but, at the expense of using a large amount of steam for stripping and dehydration. The steam explosion step, 102, may include a continuously operated pressurised reactor 218. A continuously operated steam explosion reactor has a great advantage in comparison to batch reactor(s) since the operating conditions in it does not change with time. The operating pressure in the steam explosion reactor is constant providing a continuous flow of blow-steam (b) which greatly helps recovery of heat and organic products from a steam explosion plant.

The present invention for efficient recovery of energy and chemicals from a steam explosion process is outlined schematically in area 300 of Figure 2. Blow steam (b) is first condensed with a heat-exchanger in a reboiler system 300a. The heat of condensation is used for re-boiling steam 321 from a flow of recycled and cleaned condensate 322, from a fractionation system 300b. This type of heat exchanger, where condensing heat is transferred to boiling heat is denoted "reboiler". Blow steam condensate, 333, obtained from the reboiler system 300a is contaminated with organic matter and it is sent to a fractionation system 300b for recovery of furfural (j). Cleaned condensate from the fractionation system 300b is used as feed water to the reboiler 300a for production of low pressure steam to the fractionation system 300b. It is a great advantage to use reboiled steam instead of directly using blow-steam in the fractionation system. The efficiency of fractionation system will be higher since reboiled steam has a very low concentration of volatile organic compounds, the reboiled steam is practically pure steam. Details of the fractionation system 300b are not shown in Figure 2, but it comprises one or more stripping and rectification columns which will be described in further detail below with reference to Figure 3a. The fractionation system 300b is connected to a reflux condensing system 300c. Vapours rich in furfural and other low-boilers are withdrawn from the fractionation column(s) of the fractionation system 300b to the reflux condensing system for providing reflux to said column(s).Heat is recovered with one or more reflux condensers 304, 312 provided in the reflux condensing system 300c, for re-heating a fluid return (i) for biomass drying, 101. Inert gas (k), furfural free effluent (I) and some furfural derived polymers (m) are withdrawn from the fractionation system 300b for further treatment or disposal. The present method provides in comparison to known art very efficient use of recovered energy. Waste heat (blow-steam) from a steam exploded pellet plant, 200, is used first for recovery and rectification of furfural and then for drying of biomass. Latent heat of condensation of the blow-steam is used twice, first for fractionation of contaminated condensate for recovery of furfural and then for biomass drying. In other words, one could say that the energy cost for furfural recovery is close to zero as blow-steam energy is utilized en route to biomass drying.

Blow steam (b) is contaminated with volatile organic material but possibly also with solid material, such as wood fibre residue and lignin, which may foul the heat transfer surfaces of a heat exchanger or trays or packings of a fractionation column. Typically, only one of the heat-transfer surfaces (sides) of a heat-exchanger is easily accessible for mechanical cleaning, with high pressure water or alike, while the other is not. For example, the tube-side of a tube and shell heat-exchanger is easily accessible for cleaning, but the shell side is not. The first reboiler 301 of the reboiler system 300a may be designed such that blow steam is condensed inside the tubes (if a shell and tube heat exchanger is used) and boiling of cleaned recycle condensate (i.e. particle free condensate) is on the shell side. The shell side which is difficult to clean operates with a cleaned, particle free fluid, so mechanical cleaning of the shell side is not required. Blow steam condensate, 333, obtained from the reboiler system 300a, may be purified from entrained fibres with a drum filter or alike prior to feeding it to the fractionation system. The reboiler, with condensing steam inside the tubes is so to speak used as a trap to catch particles to the blow steam condensate 333) from which they are separated mechanically (n).

Figure 3a shows schematically an energy recovery system 300 according to one embodiment of the invention. Blow-steam (b) from a steam exploded pellet plant 200, is transferred to a first reboiler 301 provided in the reboiler system 300a of the energy recovery system 300. The first reboiler 301 may be a shell and tube heat exchanger, but other types of surface heat exchangers may be used as well, for example heat exchangers furnished with lamellas or alike. Blow-steam, contaminated with volatile organic matter, wood residues and small lignin particles is condensed inside the tubes, for example such that steam enters the tubes from bottom and uncondensed steam is withdrawn from the top. It is beneficial to do so because the condensate formed will flow counter-currently downwards on the tube inner surface continuously flushing it from entrained particles and other material which may foul it. One preferred reboiler type is a "falling film reboiler". In such a reboiler is blow-steam condensed inside the heat exchanger tubes (or lamellas or alike) and the outer surface of the tubing is irrigated with feed-water which drains down as a "film on the tube surfaces" from which recycled condensate evaporates as steam. Uncondensed steam and inert gas (k) is withdrawn from the first reboiler 301 with a vacuum-pump 316 through a relief valve 318. Cleaned return condensate, 322, (returned from one or more fractionation devices in the fractionation system 300b) is re-boiled to low-pressure steam 321, with the condensing heat transferred in the first reboiler 301. Blow-steam condensate, contaminated with furfural and other organic matter is transferred with a pump 309 through a conduit 333 to the fractionation system 300b, in this embodiment to an azeotropic distillation column 303 of the fractionation system 300b. A particle filter, 339, may be installed in the conduit, 333, for removal of solid material (n) which could block trays or packings in a stripping section of column 303.

Furfural and water forms at rectification a heterogenous azeotrope at temperatures approximately below 120 °C, above this temperature is a homogenous azeotrope present. As said above, the blow-tank pressure is normally close to atmospheric pressure. The condensing temperature of blow-steam is around 100°C since (water) steam is the vast major fraction of the vapour phase. Hence, the temperature levels in the fractionation system will be below 100°C and furfural water mixtures will then exhibit heterogeneous azeotropic properties. The azeotropic concentration (with water) is around 35 weight-%. The blow-steam condensate, 333, is very diluted in furfural, in the range 1- 4 weight-%. Furfural is rectified close to the azeotropic concentration in the distillation column 303 and is withdrawn as an overhead vapour to a reflux condenser 304 provided in the reflux condensing system 300c. The reflux condensate from the reflux condenser 304 is transferred to a gravimetric decanter 305 provided in the fractionation system 300b, which gravimetric decanter 305 separates a furfural phase 334 for dehydration in a second column 311. The water phase which contains some furfural is returned as reflux 335 to the distillation column 303. The lower part of the distillation column 303 comprises a stripping section. The stripping section is heated with low-pressure steam from the first reboiler 301 such that low boilers and furfural are desorbed with high efficiency from the blow-steam condensate. The bottoms (m), from the distillation column 303 contains acetic acid, formic acid and other carboxylic acids which are transferred with a pump, 317 to an effluent cleaning plant, to biogas production or to a plant for recovery of said acids if feasible.

Low boilers, 337, mainly methanol, are withdrawn from the reflux condenser 304 with the vacuum pump 316 to a tail gas (k) handling system. The low boiler relief contains furfural as well, which may be, if feasible, recovered by installation of a methanol column in the conduit 337 (not shown in Figure 3a). Part of the cleaned (i.e. furfural free) bottoms from distillation column 303 is recycled with pump 317 to the first reboiler 301 to be used as feed water for production of low-pressure steam 321. Another aspect of the present invention is that the azeotropic distillation column is operating with a very high reflux rate. A high reflux rate is typically un-economical in fractionation due to high cost of operation (high steam usage). In the present invention the high reflux rate is not a problem, since most of the heat is recovered in the reflux condenser 304 and then used for biomass drying. An advantage of high reflux rate is that a less number of theoretical stages is required in the column 303, the column will be lower and cheaper in comparison to a standard column with an optimised (small) reflux rate. The maintenance cost and cleaning cost of a short column (low number of stages) is lower than for a tall column (large number of stages). The reflux condensate from the reflux condenser 304 is transferred to a decanter, 305. The furfural phase (heavy phase) from the decanter 305 is neutralised by addition of an alkaline chemical and pumped as a reflux to the second column 311, which may be an azeotropic dehydration column 311. The azeotrope is in this embodiment withdrawn from the top of the second column 311 to a second reflux condenser 312 and water is separated from the reflux with the decanter 305. Furfural (j) is withdrawn from a lower part of the second column 311 to a product condenser 315. Energy to the second column 311 is supplied with a second reboiler 313 energized with low-pressure steam from the first reboiler 301. The second column 311, which may be a dehydration column, has a short stripping section in the bottom. Bottoms from the second column 311 contains various furfural derived condensation products, these polymers (I) are withdrawn with a pump 314 for disposal. Steam condensate from the second reboiler 313 is collected and recycled back to the first reboiler 301 as feed water for production of low-pressure steam. It is an aspect of the invention that practically all energy in the blow steam (b) to drying passes en route over the furfural recovery. Energy in the blow steam (b) is first used for fractionation and then used for heating the return fluid (i) from the biomass dryer to high temperature and then sent back (h) for biomass drying. This way is the operational cost of furfural recovery extremely low. Another aspect is that use of a first reboiler, 301, with condensation in-side the tubes provides high operational availably when contaminated blow steam is used in the fractionation process. A third aspect is that the high reflux rate in the azeotropic distillation column 303 provides a compact design with a small number of theoretical stages resulting in an uncomplicated design with low column pressure drop and a reduced number of trays which may foul.

Figure 3b is a schematic representation of an energy recovery system 300' according to another embodiment of the invention. This embodiment is like the embodiment described in relation to Figure 3a. Most of the details are the same and are given the same or similar reference numbers and are not described again. However, the fractionation system 300b' comprises less parts. A further fractionation unit 403 is provided which receives reflux condensate from the reflux condensation system 300c. The further fractionation unit 403 may comprise one or more of the parts described in the fractionation system 300b of Figure 3a, such as an azeotropic distillation column 303 including a stripping and a rectifying section, a dehydration column 311, a decanter 305 and a reboiler 313. Furthermore, the reflux condensing system 300c' comprises only one reflux condenser 304 in this embodiment. The outline fractionation process is an example and it is possible to design it differently, with less or more columns, decanters and condensers and so on. The liquefied furfural may for example be obtained as a 35 weight-% azeotrope from the azeotropic distillation column, as 80-90 weight-% solution from the gravimetric decanter or as a product with at least 94 weight-% concentration from the dehydration column, still providing the benefit of recovering heat from overhead vapour condensing to drying.

The process and system outlined in Figures 1-4 is an example and many variations may be described without depriving the key point of the present invention, the use of waste heat contained in blow-steam first for fractionation and then for drying thus providing high energy savings in a steam exploded pellet plant furnished with furfural recovery. For example, a close related application of the present invention is to apply it to a "biorefinery" where steam explosion reactors are used for hydrolysis of biomass sugars for production of ethanol, various ethylene-glycols and alike. Blow steam from such processes contains the same hydrocarbons as mentioned above, including furfural. The energy in blow-steam can be used in the same way as described above for fractionation and heat recovered from fractionation may be used in a biorefinery perhaps not for drying, but for example for evaporation of waste liquids, preheating of process fluids, condensate stripping and like. Steam explosion processes may also produce a pressurised blow-steam (blow steam pressure above atmospheric pressure), present invention may easily be applied to such pressurised blow-steam as well. The invented method, where blow-steam energy is used two times is always applicable when final energy user provides a heat sink at a low temperature level. For example, in the case of a steam exploded pellet plant is the blow-steam temperature level typically at least 100 °C while the ambient drying air to the biomass dryer process is perhaps 10 °C and the resulting temperature driving force of the process is 90 °C. The heat sink in a biorefinery could be for example a process fluid in an evaporation process for example at 40-50 °C providing 50-60 °C driving force.

## Claims

1. A method for recovery of energy from a continuously operated steam explosion reactor (218) for production of biomass pellets or briquettes, said method comprising the steps of:
- condensing a blow-steam from the steam explosion reactor (218) for receiving a blow-steam condensate;
- recovering heat from said condensation of the blow-steam to energise a fractionation system (300b), in which fractionation system volatile hydrocarbon compounds are recovered from said blow-steam condensate; and
- recovering heat from said fractionation system (300b) for drying of biomass used in the steam explosion reactor (218).

2. Method according to claim 1, wherein the step of condensing a blow-steam is performed in a reboiler system (300a) whereby a blow-steam condensate is achieved in the reboiler system (300a) and a reboiled steam is raised in the reboiler system (300a), wherein the step of recovering heat from said condensation to energise a fractionation system (300b) comprises using said reboiled steam to energise said fractionation system (300b) and wherein said step of recovering heat from said fractionation system (300b) for drying of biomass comprises transferring an overhead vapour which is obtained in said fractionation system (300b) during recovery of volatile hydrocarbon compounds and using said overhead vapour in a reflux condensation system (300c) which is connected to the fractionation system (300b) for heating of a heat transfer fluid for drying of the biomass.

3. Method according to claim 1 or 2, further comprising the steps of:
- transferring a blow-steam (b) from a continuously operated steam explosion reactor (218) for production of biomass pellets or briquettes to a reboiler system (300a);
- condensing the blow-steam in the reboiler system (300a) for receiving a blow-steam condensate comprising water and a mixture of volatile hydrocarbon compounds comprising furfural;
- transferring the blow-steam condensate to a fractionation system (300b);
- transferring from the reboiler system (300a) to the fractionation system (300b), a reboiled steam which is raised in the reboiler system (300a) during condensation;
- using the reboiled steam to energise the fractionation system (300b) for recovery of furfural;
- transferring an overhead vapour which is obtained in the fractionation system (300b) during fractionation to a reflux condensing system (300c);
- heating a heat transfer fluid (i) in the reflux condensing system (300c) for use in a pre-heater (202) for heating of a drying gas to a biomass dryer (201) provided in the steam explosion reactor (218) for drying biomass, wherein said heating is performed by energy recovered in the reflux condensing system (300c).

4. Method according to claim 3, further comprising the step of:
- condensing in the reflux condensing system (300c) the overhead vapour to a reflux condensate comprising furfural;
- transferring said reflux condensate back to the fractionation system (300b) for further recovery of furfural.

5. Method according to any one of the claims 3-4, further comprising the step of transferring a fractionation condensate from the fractionation system (300b) to the reboiler system (300b) to be used as feed water in the reboiler system (300b), wherein said fractionation condensate is achieved in the fractionation system during recovery of furfural.

6. Method according to any one of the preceding claims, further comprising the step of withdrawing a liquefied mixture comprising furfural (j) from the fractionation system (300b), wherein the liquefied mixture (j) comprises at least 35% furfural or at least 75% furfural or at least 94 % furfural.

7. Method according to any one of the preceding claims, wherein the method is a method for recovery of energy from a process for production of pellets or briquettes from lignocellulosic biomass in a steam explosion plant (200), wherein treatment of the biomass is performed in a continuously operated reactor (218) provided in the steam explosion plant (200) under a pressure in the range 10-25 bar(a) such that the lignocellulosic biomass is heated to a temperature within 10 °C to the saturation temperature of water at the pressure prevailing in the reactor (218) and wherein the treated material and blow-steam is continuously discharged from the reactor (218) through a blow valve (221) to a blow tank (223) provided in the steam explosion plant (200).

8. Method according to any one of the preceding claims, wherein recovery of furfural in the fractionation system (300b) comprises one or more of following methods: stripping, distillation, dehydration, decanting, reflux condensing and re-boiling bottoms of fractionation columns.

9. An energy recovery system for recovery of energy from a continuously operated steam explosion reactor (218) for production of biomass pellets or briquettes, said energy recovery system (300) being configured to be connected to a steam explosion plant (200) for receiving a blow-steam (b) from a steam explosion reactor (218) provided in said steam explosion plant, said energy recovery system (300) comprising:
- a reboiler system (300a) configured to receive said blow-steam (b) from the steam explosion reactor (218), wherein said reboiler system (300a) comprises a first reboiler (301) which is configured to condense the blow-steam into a blow-steam condensate comprising water and a mixture of volatile hydrocarbon compounds comprising furfural, whereby a reboiled steam is raised in the first reboiler (301) during condensation;
- a fractionation system (300b) connected to the reboiler system (300a) and configured for receiving the blow-steam condensate and the reboiled steam from the reboiler system (300a), wherein the fractionation system (300b) further is configured to use energy from the reboiled steam for recovery of furfural from the blow-steam condensate, whereby an overhead vapour is obtained in the fractionation system (300b) during recovery of furfural; and
- a reflux condensing system (300c) connected to the fractionation system (300b) and configured for receiving the overhead vapour from the fractionation system (300b), wherein said reflux condensing system (300c) further is configured to be connected to a biomass dryer (201) provided in the steam explosion plant (200) and wherein the reflux condensing system (300c) is configured to recover energy from the overhead vapour and use this energy for heating a heat transfer fluid (i) used in the dryer (201) for drying biomass used in the steam explosion reactor (218).

10. An energy recovery system for recovery of energy from a continuously operated steam explosion reactor, said energy recovery system (300) being configured to be connected to a biorefinery for receiving a blow-steam (b) from a steam explosion reactor (218) provided in said biorefinery, said energy recovery system (300) comprising:
- a reboiler system (300a) configured to receive said blow-steam (b) from the steam explosion reactor, wherein said reboiler system (300a) comprises a first reboiler (301) which is configured to condense the blow-steam into a blow-steam condensate comprising water and a mixture of volatile hydrocarbon compounds comprising furfural, whereby a reboiled steam is raised in the first reboiler (301) during condensation;
- a fractionation system (300b) connected to the reboiler system (300a) and configured for receiving the blow-steam condensate and the reboiled steam from the reboiler system (300a), wherein the fractionation system (300b) further is configured to use energy from the reboiled steam for recovery of furfural from the blow-steam condensate, whereby an overhead vapour is obtained in the fractionation system (300b) during recovery of furfural; and
- a reflux condensing system (300c) connected to the fractionation system (300b) and configured for receiving the overhead vapour from the fractionation system (300b), wherein said reflux condensing system (300c) further is configured to be connected to the biorefinery and wherein the reflux condensing system (300c) is configured to recover energy from the overhead vapour and use this energy for heating process fluids in the biorefinery.

11. Energy recovery system according to claim 9 or 10, wherein the reflux condensing system (300c) comprises at least one reflux condenser (304) which is configured to receive said overhead vapour and condense the overhead vapour to a reflux condensate comprising furfural, wherein said reflux condensing system (300c) further is configured for transferring said reflux condensate back to the fractionation system (300b) for further recovery of furfural.

12. Energy recovery system according to any one of the claims 9-11, wherein the energy recovery system is configured for recovery of energy from a continuous steam explosion process for production of pellets or briquettes from lignocellulosic biomass in a steam explosion plant (200), wherein treatment of the biomass is performed in a reactor (218) of the steam explosion plant (200) under a pressure in the range 10-25 bar(a) such that the lignocellulosic material is heated to a temperature within 10 °C to the saturation temperature of water at the pressure prevailing in the reactor (218) and wherein the treated material and blow-steam is continuously discharged from the reactor (218) through a blow valve (221) to a blow tank (223) provided in the steam explosion plant (200).

13. Energy recovery system according to any one of the claims 9-12, wherein the fractionation system (300b) comprises one or more of an azeotropic distillation column (303) including a stripping and a rectifying section, a dehydration column (311), a decanter (305) and a reboiler (313).

14. Energy recovery system according to any one of the claims 9-13, wherein said fractionation system (300b) comprises at least one second reboiler (313) which is connected to and configured to energise a dehydration column (311) comprised in the fractionation system (300b).

15. Energy recovery system according to claim 14, wherein the second reboiler (313) is connected to the first reboiler (301) and configured for utilising reboiled steam from said first reboiler (301) as heating fluid.

16. Energy recovery system according to any one of the claims 9-15, wherein said first reboiler (301) and possibly at least one second reboiler (313) provided in the fractionation system (300b) are shell and tube heat exchangers or falling film evaporators.

17. Energy recovery system according to any one of the claims 9-16, wherein the fractionation system (300b) is configured to transfer a fractionation condensate from the fractionation system (300b) to the reboiler system (300a) to be used as feed water in the reboiler system (300a), wherein said fractionation condensate is achieved in the fractionation system during recovery of furfural.

18. A method for recovery of energy from a continuously operated steam explosion reactor provided in a biorefinery, said method comprising the steps of:
- condensing a blow-steam from the steam explosion reactor for receiving a blow-steam condensate;
- recovering heat from said condensation of the blow-steam to energise a fractionation system, in which fractionation system volatile hydrocarbon compounds are recovered from said blow-steam condensate; and
- recovering heat from said fractionation system for heating process fluids in the biorefinery.

## Patentansprüche

1. Verfahren für eine Rückgewinnung von Energie aus einem kontinuierlich betriebenen Wasserdampfexplosionsreaktor (218) für eine Herstellung von Biomassepellets oder -briketts, das Verfahren umfassend die Schritte:
- Kondensieren eines Ausblasewasserdampfes aus dem Wasserdampfexplosionsreaktor (218) zum Erhalten eines Ausblasewasserdampfkondensats;
- Rückgewinnen von Wärme aus der Kondensation des Ausblasewasserdampfes, um einem Fraktionierungssystem (300b) Energie zuzuführen, wobei in dem Fraktionierungssystem flüchtige Kohlenwasserstoffverbindungen aus dem Ausblasewasserdampfkondensat rückgewonnen werden; und
- Rückgewinnen von Wärme aus dem Fraktionierungssystem (300b) zum Trocknen von Biomasse, die in dem Wasserdampfexplosionsreaktor (218) verwendet wird.

2. Verfahren nach Anspruch 1, wobei der Schritt des Kondensierens eines Ausblasewasserdampfes in einem Verdampfersystem (300a) durchgeführt wird, wodurch ein Ausblasewasserdampfkondensat in dem Verdampfersystem (300a) erzielt wird und ein verdampfter Wasserdampf in dem Verdampfersystem (300a) erzeugt wird, wobei der Schritt des Rückgewinnens von Wärme aus der Kondensation, um dem Fraktionierungssystem (300b) Energie zuzuführen, ein Verwenden des verdampften Wasserdampfs umfasst, um dem Fraktionierungssystem (300b) Energie zuzuführen, und wobei der Schritt des Rückgewinnens von Wärme aus dem Fraktionierungssystem (300b) zum Trocknen von Biomasse ein Übertragen eines Kopfproduktdampfes, der in dem Fraktionierungssystem (300b) während der Rückgewinnung flüchtiger Kohlenwasserstoffverbindungen gewonnen wird, und das Verwenden des Kopfproduktdampfes in einem Rückflusskondensationssystem (300c) umfasst, das mit dem Fraktionierungssystem (300b) zum Erwärmen eines Wärmeübertragungsfluids zum Trocknen der Biomasse verbunden ist.

3. Verfahren nach Anspruch 1 oder 2, ferner umfassend die Schritte:
- Übertragen eines Ausblasewasserdampfes (b) aus einem kontinuierlich betriebenen Wasserdampfexplosionsreaktor (218) für die Herstellung von Biomassepellets oder -briketts an ein Verdampfersystem (300a);
- Kondensieren des Ausblasewasserdampfes in dem Verdampfersystem (300a) zum Erhalten eines Ausblasewasserdampfkondensats, umfassend Wasser und eine Mischung aus flüchtigen Kohlenwasserstoffverbindungen, umfassend Furfural;
- Übertragen des Ausblasewasserdampfkondensats an ein Fraktionierungssystem (300b);
- Übertragen, von dem Verdampfersystem (300a) an das Fraktionierungssystem (300b), eines verdampften Dampfes, der in dem Verdampfersystem (300a) während der Kondensation erzeugt wird;
- Verwenden des verdampften Dampfes, um dem Fraktionierungssystem (300b) Energie zuzuführen, für die Rückgewinnung von Furfural;
- Übertragen eines Kopfproduktdampfes, der in dem Fraktionierungssystem (300b) während der Fraktionierung gewonnen wird, an ein Rückflusskondensationssystem (300c);
- Erwärmen eines Wärmeübertragungsfluids (i) in dem Rückflusskondensationssystem (300c) zur Verwendung in einem Vorwärmer (202) zum Erwärmen eines Trocknungsgases für einen Biomassetrockner (201), der in dem Wasserdampfexplosionsreaktor (218) zum Trocknen von Biomasse bereitgestellt ist, wobei das Erwärmen durch Energie durchgeführt wird, die in dem Rückflusskondensationssystem (300c) rückgewonnen wird.

4. Verfahren nach Anspruch 3, ferner umfassend den Schritt:
- Kondensieren in dem Rückflusskondensationssystem (300c) des Kopfproduktdampfes zu einem Rückflusskondensat, umfassend Furfural;
- Übertragen des Rückflusskondensats zurück an das Fraktionierungssystem (300b) für die weitere Rückgewinnung von Furfural.

5. Verfahren nach einem der Ansprüche 3 bis 4, ferner umfassend den Schritt des Übertragens eines Fraktionierungskondensats von dem Fraktionierungssystem (300b) an das Verdampfersystem (300b), um als Speisewasser in dem Verdampfersystem (300b) verwendet zu werden, wobei das Fraktionierungskondensat in dem Fraktionierungssystem während der Rückgewinnung von Furfural erzielt wird.

6. Verfahren nach einem der vorstehenden Ansprüche, ferner umfassend den Schritt eines Entnehmens einer verflüssigten Mischung, umfassend Furfural (j), aus dem Fraktionierungssystem (300b), wobei die verflüssigte Mischung (j) mindestens 35 % Furfural oder mindestens 75 % Furfural oder mindestens 94 % Furfural umfasst.

7. Verfahren nach einem der vorstehenden Ansprüche, wobei das Verfahren ein Verfahren für die Rückgewinnung von Energie aus einem Prozess für die Herstellung von Pellets oder Briketts aus lignozellulosehaltiger Biomasse in einer Wasserdampfexplosionsanlage (200) ist, wobei eine Verarbeitung der Biomasse in einem kontinuierlich betriebenen Reaktor (218), der in der Wasserdampfexplosionsanlage (200) bereitgestellt ist, unter einem Druck in dem Bereich von 10-25 bar(a) derart durchgeführt wird, dass die lignozellulosehaltige Biomasse auf eine Temperatur innerhalb von 10 °C zu der Sättigungstemperatur von Wasser bei dem Druck erwärmt wird, der in dem Reaktor (218) herrscht, und wobei das verarbeitete Material und der Ausblasewasserdampf aus dem Reaktor (218) durch ein Ausblaseventil (221) in einen Ausblasetank (223) kontinuierlich abgeführt werden, der in der Wasserdampfexplosionsanlage (200) bereitgestellt ist.

8. Verfahren nach einem der vorstehenden Ansprüche, wobei die Rückgewinnung von Furfural in dem Fraktionierungssystem (300b) eines oder mehrere der folgenden Verfahren umfasst: Austreiben, Destillation, Dehydratation, Dekantieren, Rückflusskondensation und Verdampfen eines Sumpfes von Fraktionierungssäulen.

9. Energierückgewinnungssystem für die Rückgewinnung von Energie aus einem kontinuierlich betriebenen Wasserdampfexplosionsreaktor (218) für die Herstellung von Biomassepellets oder -briketts, wobei das Energierückgewinnungssystem (300) konfiguriert ist, um mit einer Wasserdampfexplosionsanlage (200) zum Erhalten eines Ausblasewasserdampfs (b) aus einem Wasserdampfexplosionsreaktor (218) verbunden zu werden, der in der Wasserdampfexplosionsanlage bereitgestellt ist, das Energierückgewinnungssystem (300) umfassend:
- ein Verdampfersystem (300a), das konfiguriert ist, um den Ausblasewasserdampf (b) aus dem Wasserdampfexplosionsreaktor (218) aufzunehmen, wobei das Verdampfersystem (300a) einen ersten Verdampfer (301) umfasst, der konfiguriert ist, um den Ausblasewasserdampf zu einem Ausblasewasserdampfkondensat zu kondensieren, umfassend Wasser und eine Mischung aus flüchtigen Kohlenwasserstoffverbindungen, umfassend Furfural, wobei ein verdampfter Dampf während der Kondensation in dem ersten Verdampfer (301) erzeugt wird;
- ein Fraktionierungssystem (300b), das mit dem Verdampfersystem (300a) verbunden ist und zum Erhalten des Ausblasewasserdampfkondensats und des verdampften Dampfs aus dem Verdampfersystem (300a) konfiguriert ist, wobei das Fraktionierungssystem (300b) ferner konfiguriert ist, um Energie aus dem verdampften Dampf für die Rückgewinnung von Furfural aus dem Ausblasewasserdampfkondensat zu verwenden, wodurch ein Kopfproduktdampf in dem Fraktionierungssystem (300b) während der Rückgewinnung von Furfural gewonnen wird; und
- ein Rückflusskondensationssystem (300c), das mit dem Fraktionierungssystem (300b) verbunden ist und zum Erhalten des Kopfproduktdampfes aus dem Fraktionierungssystem (300b) konfiguriert ist, wobei das Rückflusskondensationssystem (300c) ferner konfiguriert ist, um mit einem Biomassetrockner (201) verbunden zu werden, der in der Wasserdampfexplosionsanlage (200) bereitgestellt ist, und wobei das Rückflusskondensationssystem (300c) konfiguriert ist, um Energie aus dem Kopfproduktdampf rückzugewinnen und diese Energie zum Erwärmen eines Wärmeübertragungsfluids (i) zu verwenden, das in dem Trockner (201) zum Trocknen von Biomasse verwendet wird, die in dem Wasserdampfexplosionsreaktor (218) verwendet wird.

10. Energierückgewinnungssystem für die Rückgewinnung von Energie aus einem kontinuierlich betriebenen Wasserdampfexplosionsreaktor, wobei das Energierückgewinnungssystem (300) konfiguriert ist, um mit einer Bioraffinerie zum Erhalten eines Ausblasewasserdampfs (b) aus einem Wasserdampfexplosionsreaktor (218) konfiguriert ist, der in der Bioraffinerie bereitgestellt ist, das Energierückgewinnungssystem (300) umfassend:
- ein Verdampfersystem (300a), das konfiguriert ist, um den Ausblasewasserdampf (b) aus dem Wasserdampfexplosionsreaktor aufzunehmen, wobei das Verdampfersystem (300a) einen ersten Verdampfer (301) umfasst, der konfiguriert ist, um den Ausblasewasserdampf zu einem Ausblasewasserdampfkondensat zu kondensieren, umfassend Wasser und eine Mischung aus flüchtigen Kohlenwasserstoffverbindungen, umfassend Furfural, wobei ein verdampfter Dampf während der Kondensation in dem ersten Verdampfer (301) erzeugt wird;
- ein Fraktionierungssystem (300b), das mit dem Verdampfersystem (300a) verbunden ist und zum Erhalten des Ausblasewasserdampfkondensats und des verdampften Dampfs aus dem Verdampfersystem (300a) konfiguriert ist, wobei das Fraktionierungssystem (300b) ferner konfiguriert ist, um Energie aus dem verdampften Dampf für die Rückgewinnung von Furfural aus dem Ausblasewasserdampfkondensat zu verwenden, wodurch ein Kopfproduktdampf in dem Fraktionierungssystem (300b) während der Rückgewinnung von Furfural gewonnen wird; und
- ein Rückflusskondensationssystem (300c), das mit dem Fraktionierungssystem (300b) verbunden ist und zum Erhalten des Kopfproduktdampfes aus dem Fraktionierungssystem (300b) konfiguriert ist, wobei das Rückflusskondensationssystem (300c) ferner konfiguriert ist, um mit der Bioraffinerie verbunden zu werden, und wobei das Rückflusskondensationssystem (300c) konfiguriert ist, um Energie aus dem Kopfproduktdampf rückzugewinnen und diese Energie zum Erwärmen von Prozessfluiden in der Bioraffinerie zu verwenden.

11. Energierückgewinnungssystem nach Anspruch 9 oder 10, wobei das Rückflusskondensationssystem (300c) mindestens einen Rückflusskondensator (304) umfasst, der konfiguriert ist, um den Kopfproduktdampf aufzunehmen und den Kopfproduktdampf zu einem Rückflusskondensat zu kondensieren, umfassend Furfural, wobei das Rückflusskondensationssystem (300c) ferner zum Übertragen des Rückflusskondensats zurück an das Fraktionierungssystem (300b) für die weitere Rückgewinnung von Furfural konfiguriert ist.

12. Energierückgewinnungssystem nach einem der Ansprüche 9 bis 11, wobei das Energierückgewinnungssystem für die Rückgewinnung von Energie aus einem kontinuierlichen Wasserdampfexplosionsprozess für die Herstellung von Pellets oder Briketts aus lignozellulosehaltiger Biomasse in einer Wasserdampfexplosionsanlage (200) konfiguriert ist, wobei die Verarbeitung der Biomasse in einem Reaktor (218) der Wasserdampfexplosionsanlage (200) unter einem Druck in dem Bereich von 10 bis 25 bar(a) derart durchgeführt wird, dass das lignozellulosehaltige Material auf eine Temperatur innerhalb von 10 °C zu der Sättigungstemperatur von Wasser bei dem Druck erwärmt wird, der in dem Reaktor (218) herrscht, und wobei das verarbeitete Material und der Ausblasewasserdampf aus dem Reaktor (218) durch ein Ausblaseventil (221) in einen Ausblasetank (223) kontinuierlich abgeführt werden, der in der Wasserdampfexplosionsanlage (200) bereitgestellt ist.

13. Energierückgewinnungssystem nach einem der Ansprüche 9 bis 12, wobei das Fraktionierungssystem (300b) eines oder mehrere einer azeotropen Destillationskolonne (303) einschließlich eines Austreibungs- und eines Rektifikationsabschnitts, einer Dehydratationssäule (311), eines Dekanters (305) und eines Verdampfers (313) umfasst.

14. Energierückgewinnungssystem nach einem der Ansprüche 9 bis 13, wobei das Fraktionierungssystem (300b) mindestens einen zweiten Verdampfer (313) umfasst, der mit einer Dehydratationssäule (311), die in dem Fraktionierungssystem (300b) enthalten ist, verbunden ist und konfiguriert ist, um dieser Energie zuzuführen.

15. Energierückgewinnungssystem nach Anspruch 14, wobei der zweite Verdampfer (313) mit dem ersten Verdampfer (301) verbunden ist und zum Nutzen von verdampftem Dampf aus dem ersten Verdampfer (301) als Erwärmungsfluid konfiguriert ist.

16. Energierückgewinnungssystem nach einem der Ansprüche 9 bis 15, wobei der erste Verdampfer (301) und möglicherweise mindestens ein zweiter Verdampfer (313), die in dem Fraktionierungssystem (300b) bereitgestellt sind, Rohrbündelwärmetauscher oder Fallfilmverdampfer sind.

17. Energierückgewinnungssystem nach einem der Ansprüche 9 bis 16, wobei das Fraktionierungssystem (300b) konfiguriert ist, um ein Fraktionierungskondensat von dem Fraktionierungssystem (300b) an das Verdampfersystem (300a) zu übertragen, um als Speisewasser in dem Verdampfersystem (300a) verwendet zu werden, wobei das Fraktionierungskondensat in dem Fraktionierungssystem während der Rückgewinnung von Furfural erzielt wird.

18. Verfahren für die Rückgewinnung von Energie aus einem kontinuierlich betriebenen Wasserdampfexplosionsreaktor, der in einer Bioraffinerie bereitgestellt ist, das Verfahren umfassend die Schritte:
- Kondensieren eines Ausblasewasserdampfes aus dem Wasserdampfexplosionsreaktor zum Erhalten eines Ausblasewasserdampfkondensats;
- Rückgewinnen von Wärme aus der Kondensation des Ausblasedampfes, um einem Fraktionierungssystem Energie zuzuführen, wobei in dem Fraktionierungssystem flüchtige Kohlenwasserstoffverbindungen aus dem Ausblasedampfkondensat rückgewonnen werden; und
- Rückgewinnen von Wärme aus dem Fraktionierungssystem zum Erwärmen von Prozessflüssigkeiten in der Bioraffinerie.

## Revendications

1. Procédé de récupération d'énergie à partir d'un réacteur de vapocraquage (218) exploité en continu pour la production de granulés ou de briquettes de biomasse, ledit procédé comprenant les étapes consistant à :
- condenser une vapeur de soufflage provenant du réacteur de vapocraquage (218) afin de recevoir un condensat de vapeur de soufflage ;
- récupérer de la chaleur à partir de ladite condensation de la vapeur de soufflage pour alimenter en énergie un système de fractionnement (300b), dans lequel des composés hydrocarbures volatils du système de fractionnement sont récupérés à partir dudit condensat de vapeur de soufflage ; et
- récupérer de la chaleur à partir dudit système de fractionnement (300b) afin de sécher la biomasse utilisée dans le réacteur de vapocraquage (218).

2. Procédé selon la revendication 1, dans lequel l'étape de condensation d'une vapeur de soufflage est réalisée dans un système de rebouilleur (300a), moyennant quoi un condensat de vapeur de soufflage est obtenu dans le système de rebouilleur (300a) et une vapeur rebouillie est émise dans le système de rebouilleur (300a), dans lequel l'étape de récupération de chaleur à partir de ladite condensation pour alimenter en énergie un système de fractionnement (300b) comprend l'utilisation de ladite vapeur rebouillie pour alimenter en énergie ledit système de fractionnement (300b) et dans lequel ladite étape de récupération de chaleur à partir dudit système de fractionnement (300b) afin de sécher de la biomasse comprend le transfert d'une vapeur de tête qui est obtenue dans ledit système de fractionnement (300b) pendant la récupération de composés hydrocarbures volatils et l'utilisation de ladite vapeur de tête dans un système de condensation à reflux (300c) qui est relié au système de fractionnement (300b) afin de chauffer un fluide caloporteur afin de sécher la biomasse.

3. Procédé selon la revendication 1 ou 2, comprenant, en outre, les étapes consistant à :
- transférer une vapeur de soufflage (b) d'un réacteur de vapocraquage (218) exploité en continu pour la production de granulés ou de briquettes de biomasse vers un système de rebouilleur (300a) ;
- condenser la vapeur de soufflage dans le système de rebouilleur (300a) afin de recevoir un condensat de vapeur de soufflage comprenant de l'eau et un mélange de composés hydrocarbures volatils comprenant du furfural ;
- transférer le condensat de vapeur de soufflage vers un système de fractionnement (300b) ;
- transférer, du système de rebouilleur (300a) au système de fractionnement (300b), une vapeur rebouillie qui est émise dans le système de rebouilleur (300a) pendant la condensation ;
- utiliser la vapeur rebouillie pour alimenter en énergie le système de fractionnement (300b) afin de récupérer du furfural ;
- transférer une vapeur de tête qui est obtenue dans le système de fractionnement (300b) pendant le fractionnement vers un système de condensation à reflux (300c) ;
- chauffer un fluide caloporteur (i) dans le système de condensation à reflux (300c) destiné à être utilisé dans un préchauffeur (202) afin de chauffer un gaz de séchage destiné à un sécheur de biomasse (201) situé dans le réacteur de vapocraquage (218) afin de sécher de la biomasse, dans lequel ledit chauffage est réalisé par de l'énergie récupérée dans le système de condensation à reflux (300c).

4. Procédé selon la revendication 3, comprenant, en outre, l'étape consistant à :
- condenser dans le système de condensation à reflux (300c) la vapeur de tête en un condensat de reflux comprenant du furfural ;
- renvoyer ledit condensat de reflux vers le système de fractionnement (300b) en vue d'une récupération supplémentaire de furfural.

5. Procédé selon l'une quelconque des revendications 3 à 4, comprenant en outre l'étape consistant à transférer un condensat de fractionnement du système de fractionnement (300b) au système de rebouilleur (300b) pour l'utiliser en tant qu'eau d'alimentation dans le système de rebouilleur (300b), dans lequel ledit condensat de fractionnement est obtenu dans le système de fractionnement pendant la récupération de furfural.

6. Procédé selon l'une quelconque des revendications précédentes, comprenant, en outre, l'étape consistant à soutirer du système de fractionnement (300b) un mélange liquéfié comprenant du furfural (j), dans lequel le mélange liquéfié (j) comprend au moins 35 % de furfural ou au moins 75 % de furfural ou au moins 94 % de furfural.

7. Procédé selon l'une quelconque des revendications précédentes, dans lequel le procédé est un procédé de récupération d'énergie à partir d'un processus de production de granulés ou de briquettes à partir de biomasse lignocellulosique dans une installation de vapocraquage (200), dans lequel le traitement de la biomasse est effectué dans un réacteur (218) exploité en continu situé dans l'installation de vapocraquage (200) sous une pression dans la plage de 10 à 25 bar(a), de sorte que la biomasse lignocellulosique est chauffée à une température à plus ou moins 10 °C de la température de saturation de l'eau à la pression régnant dans le réacteur (218), et dans lequel le matériau traité et de la vapeur de soufflage sont évacués en continu du réacteur (218) par une vanne de soufflage (221) vers un réservoir de soufflage (223) situé dans l'installation de vapocraquage (200).

8. Procédé selon l'une quelconque des revendications précédentes, dans lequel la récupération de furfural dans le système de fractionnement (300b) comprend un ou plusieurs parmi les procédés suivants : stripping, distillation, déshydratation, décantation, condensation à reflux et rebouillage des fonds de colonnes de fractionnement.

9. Système de récupération d'énergie destiné à récupérer de l'énergie à partir d'un réacteur de vapocraquage (218) exploité en continu pour la production de granulés ou de briquettes de biomasse, ledit système de récupération d'énergie (300) étant conçu pour être relié à une installation de vapocraquage (200) afin de recevoir une vapeur de soufflage (b) provenant d'un réacteur de vapocraquage (218) situé dans ladite installation de vapocraquage, ledit système de récupération d'énergie (300) comprenant :
- un système de rebouilleur (300a) conçu pour recevoir ladite vapeur de soufflage (b) provenant du réacteur de vapocraquage (218), dans lequel ledit système de rebouilleur (300a) comprend un premier rebouilleur (301) qui est conçu pour condenser la vapeur de soufflage en un condensat de vapeur de soufflage comprenant de l'eau et un mélange de composés hydrocarbures volatils comprenant du furfural, moyennant quoi une vapeur rebouillie est émise dans le premier rebouilleur (301) pendant la condensation ;
- un système de fractionnement (300b) relié au système de rebouilleur (300a) et conçu pour recevoir le condensat de vapeur de soufflage et la vapeur rebouillie provenant du système de rebouilleur (300a), dans lequel le système de fractionnement (300b) est en outre conçu pour utiliser de l'énergie provenant de la vapeur rebouillie afin de récupérer du furfural à partir du condensat de vapeur de soufflage, moyennant quoi une vapeur de tête est obtenue dans le système de fractionnement (300b) pendant la récupération du furfural ; et
- un système de condensation à reflux (300c) relié au système de fractionnement (300b) et conçu pour recevoir la vapeur de tête provenant du système de fractionnement (300b), dans lequel ledit système de condensation à reflux (300c) est en outre conçu pour être relié à un sécheur de biomasse (201) situé dans l'installation de vapocraquage (200) et dans lequel le système de condensation à reflux (300c) est conçu pour récupérer de l'énergie à partir de la vapeur de tête et utiliser cette énergie pour chauffer un fluide caloporteur (i) utilisé dans le sécheur (201) afin de sécher de la biomasse utilisée dans le réacteur de vapocraquage (218).

10. Système de récupération d'énergie destiné à récupérer de l'énergie à partir d'un réacteur de vapocraquage exploité en continu, ledit système de récupération d'énergie (300) étant conçu pour être relié à une bioraffinerie afin de recevoir une vapeur de soufflage (b) provenant d'un réacteur de vapocraquage (218) situé dans ladite bioraffinerie, ledit système de récupération d'énergie (300) comprenant :
- un système de rebouilleur (300a) conçu pour recevoir ladite vapeur de soufflage (b) provenant du réacteur de vapocraquage, dans lequel ledit système de rebouilleur (300a) comprend un premier rebouilleur (301) qui est conçu pour condenser la vapeur de soufflage en un condensat de vapeur de soufflage comprenant de l'eau et un mélange de composés hydrocarbures volatils comprenant du furfural, moyennant quoi une vapeur rebouillie est émise dans le premier rebouilleur (301) pendant la condensation ;
- un système de fractionnement (300b) relié au système de rebouilleur (300a) et conçu pour recevoir le condensat de vapeur de soufflage et la vapeur rebouillie provenant du système de rebouilleur (300a), dans lequel le système de fractionnement (300b) est en outre conçu pour utiliser de l'énergie de la vapeur rebouillie afin de récupérer du furfural à partir du condensat de vapeur de soufflage, moyennant quoi une vapeur de tête est obtenue dans le système de fractionnement (300b) pendant la récupération du furfural ; et
- un système de condensation à reflux (300c) relié au système de fractionnement (300b) et conçu pour recevoir la vapeur de tête provenant du système de fractionnement (300b), dans lequel ledit système de condensation à reflux (300c) est en outre conçu pour être relié à la bioraffinerie et dans lequel le système de condensation à reflux (300c) est conçu pour récupérer de l'énergie à partir de la vapeur de tête et utiliser cette énergie pour chauffer des fluides de traitement dans la bioraffinerie.

11. Système de récupération d'énergie selon la revendication 9 ou 10, dans lequel le système de condensation à reflux (300c) comprend au moins un condenseur à reflux (304) qui est conçu pour recevoir ladite vapeur de tête et condenser la vapeur de tête en un condensat de reflux comprenant du furfural, dans lequel ledit système de condensation à reflux (300c) est en outre conçu pour renvoyer ledit condensat de reflux vers le système de fractionnement (300b) en vue d'une récupération supplémentaire de furfural.

12. Système de récupération d'énergie selon l'une quelconque des revendications 9 à 11, dans lequel le système de récupération d'énergie est conçu pour la récupération d'énergie à partir d'un processus de vapocraquage en continu pour la production de granulés ou de briquettes à partir de biomasse lignocellulosique dans une installation de vapocraquage (200), dans lequel un traitement de la biomasse est effectué dans un réacteur (218) de l'installation de vapocraquage (200) sous une pression dans la plage de 10 à 25 bar(a), de sorte que la matière lignocellulosique est chauffée à une température à plus ou moins 10 °C de la température de saturation de l'eau à la pression régnant dans le réacteur (218), et dans lequel la matière traitée et la vapeur de soufflage sont évacuées en continu du réacteur (218) par une vanne de soufflage (221) vers un réservoir de soufflage (223) situé dans l'installation de vapocraquage (200).

13. Système de récupération d'énergie selon l'une quelconque des revendications 9 à 12, dans lequel le système de fractionnement (300b) comprend un ou plusieurs parmi une colonne de distillation azéotropique (303) comportant une section de stripping et une section de rectification, une colonne de déshydratation (311), un décanteur (305) et un rebouilleur (313).

14. Système de récupération d'énergie selon l'une quelconque des revendications 9 à 13, dans lequel ledit système de fractionnement (300b) comprend au moins un second rebouilleur (313) qui est relié à, et conçu pour alimenter en énergie, une colonne de déshydratation (311) comprise dans le système de fractionnement (300b).

15. Système de récupération d'énergie selon la revendication 14, dans lequel le second rebouilleur (313) est relié au premier rebouilleur (301) et conçu pour utiliser de la vapeur rebouillie dudit premier rebouilleur (301) en tant que fluide de chauffage.

16. Système de récupération d'énergie selon l'une quelconque des revendications 9 à 15, dans lequel ledit premier rebouilleur (301) et éventuellement au moins un second rebouilleur (313) situé(s) dans le système de fractionnement (300b) sont des échangeurs de chaleur multitubulaires à calandre ou des évaporateurs à film descendant.

17. Système de récupération d'énergie selon l'une quelconque des revendications 9 à 16, dans lequel le système de fractionnement (300b) est conçu pour transférer un condensat de fractionnement du système de fractionnement (300b) au système de rebouilleur (300a) pour être utilisé en tant qu'eau d'alimentation dans le système de rebouilleur (300a), dans lequel ledit condensat de fractionnement est obtenu dans le système de fractionnement pendant la récupération de furfural.

18. Procédé de récupération d'énergie d'un réacteur de vapocraquage exploité en continu situé dans une bioraffinerie, ledit procédé comprenant les étapes consistant à :
- condenser une vapeur de soufflage provenant du réacteur de vapocraquage afin de recevoir un condensat de vapeur de soufflage ;
- récupérer de la chaleur à partir de ladite condensation de la vapeur de soufflage pour alimenter en énergie un système de fractionnement, dans lequel des composés hydrocarbures volatils du système de fractionnement sont récupérés à partir dudit condensat de vapeur de soufflage ; et
- récupérer de la chaleur à partir dudit système de fractionnement afin de chauffer des fluides de traitement dans la bioraffinerie.
